(19)

Europäisches Patentamt
European Patent Office
Office européen des brevets

(11) **EP 1 556 337 B1**

(12) **EUROPÄISCHE PATENTSCHRIFT**

(45) Veröffentlichungstag und Bekanntmachung des Hinweises auf die Patenterteilung:
**20.08.2014  Patentblatt 2014/34**

(21) Anmeldenummer: 03753552.3

(22) Anmeldetag: **14.10.2003**

(51) Int Cl.:
*C07C 253/24* (2006.01)    *B01J 23/00* (2006.01)
*B01J 23/20* (2006.01)    *C07C 51/215* (2006.01)

(86) Internationale Anmeldenummer:
**PCT/EP2003/011367**

(87) Internationale Veröffentlichungsnummer:
**WO 2004/035528 (29.04.2004 Gazette 2004/18)**

(54) **VERFAHREN ZUR HERSTELLUNG EINER MULTIMETALLOXIDMASSE**

METHOD FOR THE PRODUCTION OF A MULTI-METAL OXIDE MATERIAL

PROCEDE DE PRODUCTION D'UNE MASSE CONSTITUEE D'OXYDES POLYMETALLIQUES

(84) Benannte Vertragsstaaten:
**AT BE BG CH CY CZ DE DK EE ES FI FR GB GR HU IE IT LI LU MC NL PT RO SE SI SK TR**

(30) Priorität: 17.10.2002  DE 10248584
20.11.2002  DE 10254278
20.11.2002  DE 10254279

(43) Veröffentlichungstag der Anmeldung:
**27.07.2005  Patentblatt 2005/30**

(73) Patentinhaber: **BASF SE**
**67056 Ludwigshafen (DE)**

(72) Erfinder:
• **BORGMEIER, Frieder**
**68163 Mannheim (DE)**
• **MÜLLER-ENGEL, Klaus, Joachim**
**76297 Stutensee (DE)**
• **HIBST, Hartmut**
**69198 Schriesheim (DE)**
• **DIETERLE, Martin**
**68167 Mannheim (DE)**

(56) Entgegenhaltungen:
**EP-A- 1 192 987    EP-A- 1 254 707**
**EP-A- 1 254 709    EP-A- 1 254 710**
**WO-A-02/06199    WO-A-02/051539**

• **WATANABE H ET AL: "New synthesis route for Mo-V-Nb-Te mixed oxides catalyst for propane ammoxidation" APPLIED CATALYSIS A: GENERAL, ELSEVIER SCIENCE, AMSTERDAM, NL, Bd. 194-195, 13. März 2000 (2000-03-13), Seiten 479-485, XP004272252 ISSN: 0926-860X**

Bemerkungen:
Die Akte enthält technische Angaben, die nach dem Eingang der Anmeldung eingereicht wurden und die nicht in dieser Patentschrift enthalten sind.

EP 1 556 337 B1

**Beschreibung**

Vorliegende Erfindung betrifft ein Verfahren zur Herstellung einer Multimetalloxidmasse M der allgemeinen Stöchiometrie

$$MoV_aM^1_bM^2_cM^3_dO_n \qquad (I),$$

mit

$M^1 =$ wenigstens eines der Elemente aus der Gruppe umfassend Te und Sb;

$M^2 =$ wenigstens eines der Elemente aus der Gruppe umfassend Nb, Ti, W, Ta und Ce;

$M^3 =$ wenigstens eines der Elemente aus der Gruppe umfassend Pb, Ni, Co, Bi, Pd, Ag, Pt, Cu, Au, Ga, Zn, Sn, In, Re, Ir, Sm, Sc, Y, Pr, Nd und Tb;

$a =$ 0,01 bis 1,

$b =$ > 0 bis 1,

$c =$ > 0 bis 1,

$d =$ > 0 bis 0,5 und

$n =$ eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2Θ) 22,2 ± 0,5° (h), 27,3 ± 0,5° (i) und 28,2 ± 0,5° (k) liegen, wobei

- der Beugungsreflex h innerhalb des Röntgendiffraktogramms der intensitätsstärkste ist, sowie eine Halbwertsbreite von höchstens 0,5° aufweist,

- die Intensität $P_i$ des Beugungsreflexes i und die Intensität $P_k$ des Beugungsreflexes k die Beziehung $0,65 \leq R \leq 0,85$ erfüllen, in der R das durch die Formel

$$R = P_i \ / \ (P_i + P_k)$$

definierte Intensitätsverhältnis ist, und

- die Halbwertsbreite des Beugungsreflexes i und des Beugungsreflexes k jeweils $\leq 1°$ beträgt,

aber keinen Beugungsreflex mit der Scheitelpunktlage 2Θ = 50,0 ± 0,3° aufweist,
bei dem man zunächst eine solche Multimetalloxidmasse M mit der Maßgabe herstellt, daß im Verlauf der Herstellung dieser Multimetalloxidmasse M keine Vorläufermultimetalloxidmasse dieser Multimetalloxidmasse M mit einer Flüssigkeit aus der Gruppe umfassend die Gruppenmitglieder organische Säuren, anorganische Säuren, Lösungen organischer Säuren, Lösungen anorganischer Säuren und Mischungen von vorgenannten Gruppenmitgliedern gewaschen wird. Außerdem betrifft die vorliegende Erfindung die Verwendung von erfindungsgemäß erhältlichen Multimetalloxidmassen M als Aktivmasse für Katalysatoren zur heterogen katalysierten Gasphasenpartialoxidation und/oder -ammoxidation von gesättigten und/oder ungesättigten Kohlenwasserstoffen.

Multimetalloxidmassen M sind bekannt (vgl. z.B. DE-A 10248584, DE-A 10119933 und DE-A 10118814). Sie eignen sich als Katalysatoren für heterogen katalysierte partielle Gasphasenoxidationen und/oder -ammoxidationen von gesättigten und ungesättigten Kohlenwasserstoffen, wie es z.B. die vorgenannten Schriften beschreiben. Wird als Kohlenwasserstoff Propan und/oder Propen verwendet, können dabei als Zielverbindungen z.B. Acrolein, Acrylsäure und/oder Acrylnitril erzeugt werden. Diese bilden bedeutende Zwischenprodukte, die z.B. zur Herstellung von Polymerisaten Verwendung finden, die z.B. als Klebstoffe eingesetzt werden können.

Die Herstellung von Multimetalloxidmassen M erfolgt gemäß den Lehren der DE-A 10248584, DE-A 10119933 und DE-A 10118814 in systematischer Weise so, daß zunächst eine von einer Multimetalloxidmasse M verschiedene Vorläuferroultimetalloxidmasse (unter diesem Begriff sollen ganz generell Multimetalloxidmassen verstanden werden, die auf dem Herstellweg zu einer Multimetalloxidmasse M vor dieser durchlaufen werden), deren Röntgendiffraktogramm bei der Scheitelpunktlage 2Θ = 50,0 ± 0,3° einen eine Nebenphase ausweisenden Beugungsreflex aufweist, erzeugt und diese Vorläuferroultimetalloxidmasse anschließend mit einer Flüssigkeit aus der Gruppe umfassend die Gruppenmitglieder organische Säuren, anorganische Säuren, Lösungen organischer Säuren, Lösungen anorganischer Säuren und Mischungen von vorgenannten Gruppenmitgliedern gewaschen wird. Das saure Waschen von Mo und V enthaltenden Multimetalloxidmassen wird in der JP-A 8-57319 auch aus Aktivierungsgründen empfohlen.

In weniger systematischer Weise sind Multimetalloxidmassen M auf direktem Weg aber auch dadurch erhältlich, daß man aus Quellen (Ausgangsverbindungen) ihrer elementaren Konstituenten ein möglichst inniges, vorzugsweise feinteiliges Trockengemisch erzeugt und dieses anschließend durch thermische Behandlung in ein aktives Multimetalloxid M überführt, ohne dass eine wie vorstehend beschrieben durchzuführende Waschung involviert ist.

Beispielhaft genannt seien das Beispiel 11 der DE-A 19835247 sowie Example 6 der EP-A 895809.

Nachteilig an solchen auf direktem Weg erhaltenen Multimetalloxidmassen M ist jedoch, daß bei ihrer Verwendung als Katalysatoren für heterogen katalysierte partielle Gasphasen-oxidationen und/oder -ammoxidationen von gesättigten und/oder ungesättigten Kohlenwasserstoffen die Selektivität der Bildung der Zielverbindung in der Regel nicht voll zu befriedigen vermag.

Die Aufgabe der vorliegenden Erfindung bestand daher darin, ein Verfahren zur Verfügung zu stellen, das diesen Nachteil von auf direktem Weg erhaltenen Multimetalloxidmassen M weitgehend mindert oder vollständig beseitigt.

Demgemäß wurde ein Verfahren zur Herstellung von Multimetalloxidmassen M, bei dem man zunächst eine solche Multimetalloxidmasse M mit der Maßgabe herstellt, daß im Verlauf der Herstellung dieser Multimetalloxidmasse M keine Vorläufermultimetalloxidmasse dieser Multimetalloxidmasse M mit einer Flüssigkeit F aus der Gruppe umfassend die Gruppenmitglieder organische Säuren, anorganische Säuren, Lösungen organischer Säuren, Lösungen anorganischer Säuren und Mischungen von vorngenannten Gruppenmitgliedern gewaschen wird, gefunden, das dadurch gekennzeichnet ist, dass die zunächst so hergestellte Multimetalloxidmasse M mit einer Flüssigkeit F aus der Gruppe umfassend die Gruppenmitglieder organische Säuren, anorganische Säuren, Lösungen organischer Säuren, Lösungen anorganischer Säuren und Mischungen von vorngenannten Gruppenmitgliedern gewaschen wird.

Der Begriff Säure meint in dieser Schrift protische Brönsted-Säuren gemäß H.R. Christen, Grundlagen der allgemeinen und anorganischen Chemie, Sauerländer Verlag, 1973.

Als erfindungsgemäß geeignete Waschflüssigkeiten F kommen z.B. organische Säuren wie Oxalsäure, Ameisensäure, Essigsäure, Zitronensäure, Weinsäure und/oder deren Lösungen, z.B. deren wäßrige Lösungen, in Betracht. Eine solche wäßrige Lösung kann auch zwei oder mehr der vorgenannten organischen Säuren gelöst enthalten. Anstelle von Wasser können als Lösungsmittel auch Alkohole (z.B. Methanol und/oder Ethanol) und/oder deren wäßrige Lösungen eingesetzt werden. Anstelle der genannten organischen Säuren können erfindungsgemäß als Waschflüssigkeit F auch anorganische Säuren wie z.B. Salpetersäure verwendet werden. Auch die anorganischen Säuren können in wässriger oder sonstiger, z.B. alkoholischer oder wäßrig-alkoholischer (z.B. methanolischer und/oder ethanolischer) Lösung eingesetzt werden. Erfindungsgemäß bevorzugt wird als Waschflüssigkeit F eine wäßrige Salpetersäure verwendet.

Selbstredend kann als Waschflüssigkeit F auch ein Gemisch aus organischen und anorganischen Säuren eingesetzt werden. Dabei kann dieses Gemisch ebenso wie alle vorstehenden Säuren in Wasser, Alkoholen (z.B. Methanol oder Ethanol) oder deren Gemischen zum Einsatz kommen. In der Regel enthalten die Säurelösungen 5 bis 30 Gew.-%, häufig 5 bis 15 Gew.-% an Säure gelöst. Bei Bedarf kann den genannten sauren Waschflüssigkeiten F zusätzlich Wasserstoffperoxid zugesetzt werden.

Die erfindungsgemäße Waschung wird mit Vorteil bei erhöhter Temperatur durchgeführt. In anwendungstechnisch zweckmäßiger Weise wird man die auf direktem Weg erhaltene Multimetalloxid-masse M (bevorzugt in feinteiliger Form) mit der Waschflüssigkeit F versetzen und einige Stunden (in der Regel 1 h bis 10 h) unter Rückfluß rühren. Dabei in typischer Weise angewendete Temperaturen betragen 50 bis 100°C. Nach beendeter Waschung wird der verbliebene Feststoff normalerweise durch Filtration von der Flüssigphase getrennt und nachfolgend, z.B. mit Wasser, vom Waschmittel frei gewaschen. Abschließend wird der gewaschene Feststoff, zweckmäßig bei erhöhter Temperatur, getrocknet.

Erfindungsgemäß bemerkenswert ist, daß die Performance von auf direktem Weg erhaltenen Multimetalloxidmassen M durch die erfindungsgemäße Verfahrensweise verbessert werden kann, obwohl sie ausweislich ihres Röntgendiffraktogramms die üblicherweise störende und in den erfindungsgemäß zu verwendenden Waschflüssigkeiten F in der Regel gut lösliche, durch ihren Beugungsreflex bei der Scheitelpunktlage $2\Theta = 50,0 \pm 0,3°$ ausgewiesene, Nebenphase nicht ersichtlich enthalten.

Dies ist eventuell darauf zurückzuführen, daß auf direktem Weg erhaltene Multimetalloxidmassen M die besagte Nebenphase nur in winzigsten, röntgenographisch kaum nachweisbaren Mengen auf ihrer Oberfläche als Belag enthalten, der durch die erfindungsgemäße Verfahrensweise abgelöst wird.

Alle in dieser Schrift auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall ($2\Theta$):0,02°, Meßzeit je Schritt: 2,4 s, Detektor: Scintillationszählrohr). Die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition; das gleiche gilt für die Definition der Halbwertsbreite.

Erfindungsgemäß bevorzugt gilt für die Multimetalloxidmasse M, sowohl vor als auch nach ihrer erfindungsge-

mäßen Waschung, $0{,}67 \leq R \leq 0{,}75$ und ganz besonders bevorzugt gilt R = 0,69 bis 0,75 bzw. R = 0,71 bis 0,74, bzw. R = 0,72.

Neben den Beugungsreflexen h, i und k enthält das Röntgendiffraktogramm von Multimetalloxidmassen M, sowohl vor als auch nach ihrer erfindungsgemäßen Waschung, in der Regel noch weitere Beugungsreflexe, deren Scheitelpunkte bei den nachfolgenden Beugungswinkeln (2Θ) liegen:

$9{,}0 \pm 0{,}4°$ (l),
$6{,}7 \pm 0{,}4°$ (o) und
$7{,}9 \pm 0{,}4°$ (p).

Günstig ist es ferner, wenn das Röntgendiffraktogramm zusätzlich einen Beugungsreflex enthält, dessen Scheitelpunkt beim Beugungswinkel (2Θ) = $45{,}2 \pm 0{,}4°$ (q) liegt.

Häufig enthält das Röntgendiffraktogramm von Multimetalloxidmassen M, sowohl vor als auch nach ihrer erfindungsgemäßen Waschung, auch noch die Reflexe $29{,}2 \pm 0{,}4°$ (m) und $35{,}4 \pm 0{,}4°$ (n) (Scheitelpunktlagen).

Ordnet man dem Beugungsreflex h die Intensität 100 zu, ist es erfindungsgemäß günstig, wenn die Beugungsreflexe i, l, m, n, o, p, q in der gleichen Intensitätsskala, sowohl vor als auch nach erfindungsgemäßer Waschung der Multimetalloxidmasse M, die nachfolgenden Intensitäten aufweisen:

i: 5 bis 95, häufig 5 bis 80, teilweise 10 bis 60;
l: 1 bis 30;
m: 1 bis 40;
n: 1 bis 40;
o: 1 bis 30;
p: 1 bis 30 und
q: 5 bis 60.

Enthält das Röntgendiffraktogramm der erfindungsgemäß erhältlichen Multimetalloxidmassen M von den vorgenannten zusätzlichen Beugungsreflexen, ist die Halbwertsbreite derselben in der Regel sowohl vor als auch nach der erfindungsgemäßen Waschung $\leq 1°$.

Die spezifische Oberfläche von erfindungsgemäß erhältlichen Multimetalloxidmassen M beträgt vielfach 1 bis 40 m$^2$/g, häufig 15 bis 40 bzw. 30 m$^2$/g (bestimmt nach der BET-Methode, Stickstoff).

Erfindungsgemäß bevorzugt beträgt der stöchiometrische Koeffizient a der erfindungsgemäß erhältlichen Multimetalloxidmassen M, unabhängig von den Vorzugsbereichen für die anderen stöchiometrischen Koeffizienten der erfindungsgemäß erhältlichen Multimetalloxidmassen M, 0,05 bis 0,6, besonders bevorzugt 0,1 bis 0,6 oder 0,5.

Unabhängig von den Vorzugsbereichen für die anderen stöchiometrischen Koeffizienten der erfindungsgemäß erhältlichen Multimetalloxidmassen M beträgt der stöchiometrische Koeffizient b bevorzugt 0,01 bis 1, und besonders bevorzugt 0,01 bzw. 0,1 bis 0,5 oder 0,4.

Der stöchiometrische Koeffizient c der erfindungsgemäß erhältlichen Multimetalloxidmassen M beträgt, unabhängig von den Vorzugsbereichen für die anderen stöchiometrischen Koeffizienten der erfindungsgemäß erhältlichen Multimetalloxidmassen M 0,01 bis 1 und besonders bevorzugt 0,01 bzw. 0,1 bis 0,5 oder 0,4. Ein ganz besonders bevorzugter Bereich für den stöchiometrischen Koeffizienten c, der, unabhängig von den Vorzugsbereichen für die anderen stöchiometrischen Koeffizienten der erfindungsgemäß erhältlichen Multimetalloxidmassen M, mit allen anderen Vorzugsbereichen in dieser Schrift kombinierbar ist, ist der Bereich 0,05 bis 0,2.

Erfindungsgemäß ist der stöchiometrische Koeffizient d der erfindungsgemäß erhältlichen Multimetalloxidmassen M, unabhängig von den Vorzugsbereichen für die anderen stöchiometrischen Koeffizienten der erfindungsgemäß erhältlichen Multimetalloxidmassen M, > 0 und beträgt vorteilhaft 0,00005 bzw. 0,0001 bis 0,5, besonders bevorzugt 0,0005 bis 0,3, häufig 0,00075 bis 0,2 und oft 0,001 bis 0,01 bzw. 0,1.

Besonders günstig sind erfindungsgemäß erhältliche Multimetalloxidmassen M, deren stöchiometrische Koeffizienten a, b, c und d gleichzeitig im nachfolgenden Raster liegen:

a = 0,05 bis 0,6;
b = 0,01 bis 1 (bzw. 0,01 bis 0,5);
c = 0,01 bis 1 (bzw. 0,01 bis 0,5); und
d = 0,0001 bis 0,5 (bzw. 0,0005 bis 0,3).

Ganz besonders günstig sind erfindungsgemäß erhältliche Multimetalloxidmassen M, deren stöchiometrische Koeffizienten a, b, c und d gleichzeitig im nachfolgenden Raster liegen:

a = 0,1 bis 0,6;

b = 0,1 bis 0,5;

c = 0,1 bis 0,5; und

d = 0,00075 bis 0,2, bzw. 0,001 bis 0,01, bzw. 0,001 bis 0,1.

$M^1$ ist bevorzugt Te.

Alles vorgenannte gilt vor allem dann, wenn in den erfindungsgemäß erhältlichen Multimetalloxidmassen M $M^2$ wenigstens zu 50 mol-% seiner Gesamtmenge Nb und ganz besonders bevorzugt dann, wenn $M^2$ zu wenigstens 75 mol-% seiner Gesamtmenge, bzw. zu 100 mol-% seiner Gesamtmenge Nb ist.

Es gilt vor allem aber auch, unabhängig von der Bedeutung von $M^2$, dann, wenn $M^3$ wenigstens ein Element aus der Gruppe umfassend Ni, Co, Bi, Pd, Ag, Au, Pb und Ga oder wenigstens ein Element aus der Gruppe umfassend Ni, Co, Pd und Bi ist.

Alles vorgenannte gilt vor allem aber auch dann, wenn $M^2$ in den erfindungsgemäß erhältlichen Multimetalloxidmassen M wenigstens zu 50 mol-% seiner Gesamtmenge, oder zu wenigstens 75 mol-%, oder zu 100 mol-% Nb und $M^3$ wenigstens ein Element aus der Gruppe umfassend Ni, Co, Bi, Pd, Ag, Au, Pb und Ga ist.

Alles vorgenannte gilt vor allem aber auch dann, wenn $M^2$ in den erfindungsgemäß erhältlichen Multimetalloxidmassen M wenigstens zu 50 mol-%, oder zu wenigstens 75 mol-%, oder zu 100 mol-% seiner Gesamtmenge Nb und $M^3$ wenigstens ein Element aus der Gruppe umfassend Ni, Co, Pd und Bi ist.

Ganz besonders bevorzugt gelten alle Aussagen hinsichtlich der stöchiometrischen Koeffizienten dann, wenn in den erfindungsgemäß erhältlichen Multimetalloxidmassen M $M^1$ = Te, $M^2$ = Nb und $M^3$ = wenigstens ein Element aus der Gruppe umfassend Ni, Co und Pd.

Weitere erfindungsgemäß geeignete Stöchiometrien für die erfindungsgemäß erhältlichen Multimetalloxidmassen M sind jene, die im eingangs zitierten Stand der Technik offenbart sind.

Erfindungsgemäß zu waschende Multimetalloxidmassen M können unter geeigneten Umständen z.B. nach den in den Schriften DE-A 19835247, EP-A 529853, EP-A 603836, EP-A 608838, EP-A 895809, DE-A 19835247, EP-A 962253, EP-A 1080784, EP-A 1090684, EP-A 1123738, EP-A 1192987, EP-A 1192986, EP-A 1192982, EP-A 1192983 und EP-A 1192988 beschriebenen Herstellverfahren erhalten werden. Nach diesen Verfahren wird von geeigneten Quellen der elementaren Konstituenten der Multimetalloxidmasse ein möglichst inniges, vorzugsweise feinteiliges, Trockengemisch erzeugt und dieses bei Temperaturen von 350 bis 700°C bzw. 400 bis 650°C oder 400 bis 600°C thermisch behandelt. Die thermische Behandlung kann prinzipiell sowohl unter oxidierender, reduzierender als auch unter inerter Atmosphäre erfolgen. Als oxidierende Atmosphäre kommt z.B. Luft, mit molekularem Sauerstoff angereicherte Luft oder an Sauerstoff entreicherte Luft in Betracht. Vorzugsweise wird die thermische Behandlung jedoch un-ter inerter Atmosphäre, d.h., z.B. unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Erfolgt die thermische Behandlung unter gasförmiger Atmosphäre, kann diese sowohl stehen als auch fließen. Vorzugsweise fließt sie. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (z.B. unter Luft) bei einer Temperatur von 150 bis 400°C bzw. 250 bis 350°C (= Vorzersetzungsschritt). Im Anschluß daran wird die thermische Behandlung zweckmäßig unter Inertgas bei Temperaturen von 350 bis 700°C bzw. 400 bis 650°C oder 450 bis 600°C fortgesetzt. Selbstredend kann die thermische Behandlung auch so erfolgen, daß die innig gemischte Trockenmasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder versplittet wird.

Das innige Vermischen der Ausgangsverbindungen kann in trockener oder in nasser Form erfolgen.

Erfolgt es in trockener Form, werden die Ausgangsverbindungen zweckmäßigerweise als feinteilige Pulver eingesetzt und,nach dem Mischen und gegebenenfalls Verdichten der Calcinierung (thermischen Behandlung) unterworfen.

Vorzugsweise erfolgt das innige Vermischen jedoch in nasser Form. Üblicherweise werden die Ausgangsverbindungen dabei in Form einer wäßrigen Lösung (gegebenenfalls unter Mitverwendung komplexbildender Mittel; vgl. z.B. DE-A 10145958) und/oder Suspension miteinander vermischt. Anschließend wird die wäßrige Masse getrocknet und nach der Trocknung calciniert. Zweckmäßigerweise handelt es sich bei der wäßrigen Masse um eine wäßrige Lösung oder um eine wäßrige Suspension. Vorzugsweise erfolgt der Trocknungsprozeß unmittelbar im Anschluß an die Herstellung der wäßrigen Mischung (insbesondere im Fall einer wäßrigen Lösung; vgl. z.B. JP-A 7-315842) und durch Sprühtrocknung (die Austrittstemperaturen betragen in der Regel 100 bis 150°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung oder Suspension handelt. Es kann aber auch durch Eindampfen im Vakuum, durch Gefriertrocknung, oder durch konventionelles Eindampfen getrocknet werden.

Als Quellen für die elementaren Konstituenten kommen im Rahmen der Durchführung der vorstehend beschriebenen Herstellweise alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/ oder Hydroxide der elementaren Konstituenten mitverwendet oder ausschließlich verwendet werden. D.h., insbesondere kommen alle in den Schriften des gewürdigten Standes der Technik genannten Ausgangsverbindungen in Betracht.

Erfindungsgemäß geeignete Quellen für das Element Mo sind z.B. Molybdänoxide wie Molybdäntrioxid, Molybdate wie Ammoniumheptamolybdattetrahydrat und Molybdänhalogenide wie Molybdänchlorid.

Geeignete, erfindungsgemäß mitzuverwendende Ausgangsverbindungen für das Element V sind z.B. Vanadiumoxysulfathydrat, Vanadylacetylacetonat, Vanadate wie Ammoniummetavanadat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Dabei können als Vanadinausgangsverbindungen auch solche mitverwendet werden, die das Vanadin in der Oxidationsstufe +4 enthalten.

Als Quellen für das Element Tellur eignen sich erfindungsgemäß Telluroxide wie Tellurdioxid, metallisches Tellur, Tellurhalogenide wie $TeCl_2$, aber auch Tellursäuren wie Orthothellursäure $H_6TeO_6$.

Vorteilhafte Antimonausgangsverbindungen sind Antimonhalogenide wie $SbCl_3$, Antimonoxide wie Antimontrioxid ($Sb_2O_3$), Antimonsäuren wie $HSb(OH)_6$, aber auch Antimonoxid-Salze wie Antimonoxid-sulfat ($SbO)_2SO_4$.

Erfindungsgemäß geeignete Niobquellen sind z. B. Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Carbonsäuren und/oder Dicarbonsäuren wie z. B. Oxalate und Alkoholate. Selbstredend kommen als Niobquelle auch die in der EP-A 895 809 verwendeten Nb enthaltenden Lösungen in Betracht.

Bezüglich aller anderen möglichen Elemente (insbesondere des Ni, Pb, Cu, Co, Bi und Pd) kommen als geeignete Ausgangsverbindungen vor allem deren Halogenide, Nitrate, Formiate, Oxalate, Acetate, Carbonate und/oder Hydroxide in Betracht. Geeignete Ausgangsverbindungen sind vielfach auch deren Oxoverbindungen wie z. B. Wolframate bzw. die von diesen abgeleiteten Säuren. Häufig werden als Ausgangsverbindungen auch Ammoniumsalze eingesetzt.

Ferner kommen als Ausgangsverbindungen auch Polyanionen vom Anderson Typ in Betracht, wie sie z. B. in Polyhedron Vol. 6, No. 2, pp. 213-218, 1987 beschrieben sind. Eine weitere geeignete Li-teraturquelle für Polyanionen vom Anderson Typ bildet Kinetics and Catalysis, Vol. 40, No. 3, 1999, pp 401 bis 404.

Andere als Ausgangsverbindungen geeignete Polyanionen sind z. B. solche vom Dawson oder Keggin Typ. Vorzugsweise werden solche Ausgangsverbindungen verwendet, die sich bei erhöhten Temperaturen entweder im Beisein oder bei Ausschluß von Sauerstoff, gegebenenfalls unter Freisetzung gasförmiger Verbindungen, in ihre Oxide umwandeln.

Die wie beschrieben auf direktem Weg erhältlichen Multimetalloxidmassen M können dann wie beschrieben durch erfindungsgemäßes Waschen in erfindungsgemäß erhältliche Multimetalloxidmassen M überführt werden.

Mit erhöhter Häufigkeit gelingt die Herstellung von erfindungsgemäß zu waschenden Multimetalloxidmassen M wenn ihre Herstellung auf hydrothermalem Weg erfolgt, wie es z.B. die DE-A 10029338 und die JP-A 2000-143244 beschreiben. Dabei wird ein Gemisch aus Quellen der elementaren Konstituenten der Multimetalloxidmasse M einer hydrothermalen Behandlung unterworfen. Der sich dabei neu bildende Feststoff wird abgetrennt und als inniges Trockengemisch wie beschrieben durch thermische Behandlung in eine Multimetalloxidmasse M überführt. Die hydrothermale Verfahrensweise ist dem Fachmann vertraut (vgl. Spalte 2, unten, der DE-A 10029338). Im besonderen wird hier darunter die thermische Behandlung eines, vorzugsweise innigen, Gemisches von Quellen der elementaren Konstituenten der gewünschten Multimetalloxidmasse M in einem Überdruckgefäß (Autoklav) im Beisein von überatmosphärischen Druck aufweisendem Wasserdampf, üblicherweise bei Temperaturen im Bereich von > 100°C bis 600°C, verstanden. Der Druchbereich erstreckt sich dabei in typischer Weise auf bis zu 500 atm, vorzugsweise auf bis zu 250 atm. Selbstverständlich können auch Temperaturen oberhalb von 600°C und Wasserdampfdrucke oberhalb von 500 atm angewendet werden, was anwendungstechnisch jedoch wenig zweckmäßig ist. Mit besonderem Vorteil erfolgt die hydrothermale Behandlung unter solchen Bedingungen, unter denen Wasserdampf und flüssiges Wasser koexistieren.

Dies ist im Temperaturbereich von > 100°C bis 374, 15°C (kritische Temperatur des Wasser) unter Anwendung der entsprechenden Drücke möglich.

Die Mengen an Wasser werden dabei zweckmäßig so bemessen, daß die flüssige Phase die Gesamtmenge der Ausgangsverbindungen in Suspension und/oder Lösung aufzunehmen vermag. Es ist aber auch eine solche Verfahrensweise möglich, bei der das innige Gemisch der Ausgangsverbindungen die mit dem Wasserdampf im Gleichgewicht befindliche flüssige Wassermenge vollständig absorbiert.

Vorteilhaft erfolgt die hydrothermale Behandlung bei Temperaturen von > 100 bis 300°C, vorzugsweise bei Temperaturen von 150 bis 250°C (z.B. 160 bis 200°C). Bezogen auf die Summe aus Wasser und Quellen der elementaren Konstituenten der gewünschten Multimetalloxidmasse M beträgt der Gewichtsanteil der letzteren im Autoklaven in der Regel wenigstens 1 Gew.-%. Üblicherweise liegt der vorgenannte Gewichtsanteil nicht oberhalb von 90 Gew.-%. Typisch sind Gewichtsanteile von 30 bis 60, bzw. von 5 bis 30 Gew.-%, häufig 5 bis 15 Gew.-%.

Während der hydrothermalen Behandlung kann sowohl gerührt als auch nicht gerührt werden. Die hydrothermale Behandlung selbst nimmt in der Regel eine Zeitdauer von wenigen Stunden bis zu einigen Tagen in Anspruch. Typisch ist ein Zeitraum von 48 h.

Anwendungstechnisch zweckmäßig ist der für die hydrothermale Behandlung zu verwendende Autoklav innenseitig mit Teflon beschichtet. Vorab der hydrothermalen Behandlung kann der Autoklav, gegebenenfalls einschließlich des enthaltenen wäßrigen Gemisches, evakuiert werden. Anschließend kann vor der Temperaturerhöhung mit Inertgas ($N_2$, Edelgas) gefüllt werden. Beide Maßnahmen können auch unterlassen werden. Selbstredend kann das wäßrige Gemisch zur Inertisierung vorab einer hydrothermalen Behandlung zusätzlich mit Inertgas gespült werden. Die vorgenannten Inertgase können anwendungstechnisch zweckmäßig auch dazu benutzt werden, bereits vorab der hydrothermalen Behandlung im Autoklaven überatmosphärischen Druck einzustellen.

Die zur Erzeugung der erfindungsgemäß zu waschenden Multimetalloxidmasse M erforderliche thermische Behandlung des sich im Verlauf der hydrothermalen Behandlung neu gebildeten und nach Beendigung der hydrothermalen Behandlung (nach Beendigung der hydrothermalen Behandlung kann der Autoklav entweder auf Raumtemperatur abgeschreckt oder langsam, d.h., über einen längeren Zeitraum (z.B. durch sich selbst überlassen) auf Raumtemperatur gebracht werden) abgetrennten Feststoffs (innigen Gemischs) wird (wie im Fall der konventionellen Herstellung bereits beschrieben) zweckmäßigerweise bei einer Temperatur von 350 bis 700°C, häufig bei einer Temperatur von 400 bis 650°C, bzw. 400 bis 600°C durchgeführt. Sie kann sowohl unter oxidierender, reduzierender als auch unter inerter Atmosphäre erfolgen. Als oxidierende Atmosphäre kommt z.B. Luft, mit molekularem Sauerstoff angereicherte Luft oder an Sauerstoff entreicherte Luft in Betracht. Vorzugsweise wird die thermische Behandlung unter inerter Atmosphäre, d.h., z.B. unter molekularem Stickstoff und/oder Edelgas, durchgeführt. Selbstverständlich kann die thermische Behandlung auch unter Vakuum erfolgen.

Die Herstellung von erfindungsgemäß zu behandelnden Multimetall-oxidmassen M kann aber auch dadurch erfolgen, dass man zunächst eine Multimetalloxidmasse M' erzeugt, die sich von einer Multimetalloxidmasse M nur dadurch unterscheidet, dass d = 0 ist. Eine solche, bevorzugt feinteilige, Multimetalloxidmasse M' kann dann mit Lösungen (z.B. wässrigen) von Elementen $M^3$ getränkt (z.B. durch Besprühen), nachfolgend getrocknet (bevorzugt bei Temperaturen ≤ 100°C) und anschließend, wie bereits beschrieben, calciniert (bevorzugt im Inertgasstrom) werden (oft wird hier auf eine Vorzersetzung an Luft verzichtet) werden. Die Verwendung von wäßrigen Nitrat- und/oder Halogenidlösungen von Elementen $M^3$ und/ oder die Verwendung von wässrigen Lösungen in denen die Elemente $M^3$ mit organischen Verbindungen (z.B. Acetate oder Acetylacetonate) komplexiert vorliegen ist für diese Herstellvariante besonders vorteilhaft.

Die durch erfindungsgemäßes Waschen erhältlichen Multimetalloxide M können als solche [z.B. als Pulver oder nach Tablettieren des Pulvers (häufig unter Zusatz von 0,5 bis 2 Gew.-% an feinteiligem Graphit) und nachfolgendem Versplitten zu Splitt zerkleinert] oder auch zu Formkörpern geformt als Katalysatoren für heterogen katalysierte Gasphasenpartialoxidationen und/oder -ammoxidationen von gesättigten und/oder ungesättigten Kohlenwasserstoffen eingesetzt werden. Dabei kann das Katalysatorbett ein Festbett, ein Wanderbett oder ein Wirbelbett sein.

Die Formung zu Formkörpern kann z.B. durch Aufbringen auf einen Trägerkörper erfolgen, wie es z.B. in der DE-A 10118814 bzw. PCT/EP/02/04073 bzw. DE-A 10051419 beschrieben wird.

Die dabei für die erfindungsgemäß erhältlichen Multimetalloxidmassen M zu verwendenden Trägerkörper sind vorzugsweise chemisch inert. D.h., sie greifen in den Ablauf der partiellen katalytischen Gasphasenoxidation bzw. -ammoxidation des Kohlenwasserstoffs (z.B. Propan und/oder Propen zu Acrylsäure), die durch die erfindungsgemäß erhältlichen Multimetalloxidmassen M katalysiert wird, im wesentlichen nicht ein.

Als Material für die Trägerkörper kommen erfindungsgemäß insbesondere Aluminiumoxid, Siliciumdioxid, Silicate wie Ton, Kaolin, Steatit (bevorzugt mit geringem in Wasser löslichem Alkaligehalt), Bims, Aluminiumsilicat und Magnesiumsilicat, Siliciumcarbid, Zirkondioxid und Thoriumdioxid in Betracht. Bevorzugt werden Träger mit minimalem bzw. verschwindendem Alkaligehalt eingesetzt.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt. Häufig liegt die Oberflächenrauhigkeit $R_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen ≤ 1 Vol.-%).

Die Dicke der auf den erfindungsgemäßen Schalenkatalysatoren befindlichen aktiven Oxidmassenschale liegt üblicherweise bei 10 bis 1000 $\mu$m. Sie kann aber auch 50 bis 700 $\mu$m, 100 bis 600 $\mu$m oder 150 bis 400 $\mu$m betragen. Mögliche Schalendicken sind auch 10 bis 500 $\mu$m, 100 bis 500 $\mu$m oder 150 bis 300 $\mu$m.

Prinzipiell kommen beliebige Geometrien der Trägerkörper in Betracht. Ihre Längstausdehnung beträgt in der Regel 1 bis 10 mm. Vorzugsweise werden jedoch Kugeln oder Zylinder, insbesondere Hohlzylinder, als Trägerkörper angewendet. Günstige Durchmesser für Trägerkugeln betragen 1,5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung von Schalenkatalysatoren kann in einfachster Weise so erfolgen, dass man erfindungsgemäß erhältliche Oxidmassen M vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse M eine Schicht der Aktivmasse auf der befeuchteten Oberfläche angeheftet. Abschließend wird der beschichtete Trägerkörper getrocknet. Selbstredend kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden erfindungsgemäß erhältlichen katalytisch aktiven Oxidmasse M wird selbstredend an die gewünschte Schalendicke angepaßt. Für den Schalendickenbereich von 100 bis 500 $\mu$m eignen sich z. B. solche Aktivmassenpulver, von denen wenigstens 50 % der Gesamtzahl der Pulverpartikel ein Sieb der Maschenweite 1 bis 20 $\mu$m passieren und deren numerischer Anteil an Partikeln mit einer Längstausdehnung oberhalb von 50 $\mu$m weniger als 10 % beträgt. In der Regel entspricht die Verteilung der Längstausdehnungen der Pulverpartikel herstellungsbedingt einer Gaußverteilung. Häufig ist die Korngrößenverteilung wie folgt beschaffen:

| D ($\mu$m) | 1 | 1,5 | 2 | 3 | 4 | 6 | 8 | 12 | 16 | 24 | 32 |
|---|---|---|---|---|---|---|---|---|---|---|---|
| x | 80,5 | 76,3 | 67,1 | 53,4 | 41,6 | 31,7 | 23 | 13,1 | 10,8 | 7,7 | 4 |
| y | 19,5 | 23,7 | 32,9 | 46,6 | 58,4 | 68,3 | 77 | 86,9 | 89,2 | 92,3 | 96 |

| D ($\mu$m) | 48 | 64 | 96 | 128 |
|---|---|---|---|---|
| x | 2,1 | 2 | 0 | 0 |
| y | 97,9 | 98 | 100 | 100 |

Dabei sind:

D = Durchmesser des Korns,
x = der prozentuale Anteil der Körner, deren Durchmesser $\geq$ D ist; und
y = der prozentuale Anteil der Körner, deren Durchmesser < D ist.

Für eine Durchführung des beschriebenen Beschichtungsverfahrens im technischen Maßstab empfiehlt sich z. B. die Anwendung des in der DE-A 2909671, sowie der in der DE-A 10051419 offenbarten Verfahrensprinzips. D.h., die zu beschichtenden Trägerkörper werden in einem vorzugsweise geneigten (der Neigungswinkel beträgt in der Regel $\geq$ 0° und $\leq$ 90°, meist $\geq$ 30° und $\leq$ 90°; der Neigungswinkel ist der Winkel der Drehbehältermittelachse gegen die Horizontale) rotierenden Drehbehälter (z. B. Drehteller oder Dragiertrommel) vorgelegt. Der rotierende Drehbehälter führt die z. B. kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (z.B. eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse M zuzuführen (z.B. über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des z. B. zylinder-oder kugelförmigen Trägerkörpers zu einer zusammenhängenden Schale verdichtet.

Bei Bedarf durchläuft der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können usw. (eine Zwischentrocknung ist in der Regel nicht erforderlich). Feinteilige oxidische Aktivmasse M und flüssiges Bindemittel werden dabei in der Regel kontinuierlich und simultan zugeführt.

Die Entfernung des flüssigen Bindemittels kann nach beendeter Beschichtung z. B. durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

Wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 2909671 und in der DE-A 10051419.

Die vorerwähnte abschließende Entfernung des verwendeten flüssigen Bindemittels kann in kontrollierter Weise z. B. durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trokkenofen beliebiger Art (z. B. Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden. Als Bindemittel für den Beschichtungsprozeß können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Selbstredend kann die Formgebung von erfindungsgemäß erhältlichen Multimetalloxidmassen M auch durch Extrusion und/oder Tablettierung von feinteiliger erfindungsgemäß erhältlicher Multimetalloxidmasse M erfolgen.

Als Geometrien kommen dabei sowohl Kugeln, Vollzylinder und Hohlzylinder (Ringe) in Betracht. Die Längstausdehnung der vorgenannten Geometrien beträgt dabei in der Regel 1 bis 10 mm. Im Fall von Zylindern beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Vollkatalysatoren können auch eine Länge von 3 bis 6 mm, einen Außendurchmesser von 4 bis 8 mm und eine Wanddicke von 1 bis 2 mm aufweisen. Möglich ist aber auch eine Vollkatalysatorringgeometrie von 7 mm x 3 mm x 4 mm oder von 5 mm x 3 mm x 2 mm (Außendurchmesser x Länge x Innendurchmesser).

Selbstredend kommen für die Geometrie der als Katalysatoren zu verwendenden erfindungsgemäß erhältlichen Multimetalloxidaktivmassen M auch all jene der DE-A 10101695 in Betracht.

Die Definition der Intensität eines Beugungsreflexes im Röntgendiffraktogramm bezieht sich in dieser Schrift, wie bereits gesagt, auf die in der DE-A 19835247, sowie die in der DE-A 10051419 und DE-A 10046672 niedergelegte Definition. D.h., bezeichnet $A^1$ den Scheitelpunkt eines Reflexes 1 und bezeichnet $B^1$ in der Linie des Röntgendiffraktogramms bei Betrachtung entlang der zur $2\Theta$-Achse senkrecht stehenden Intensitätsache das nächstliegende ausgeprägte Minimum (Reflexschultern ausweisende Minima bleiben unberücksichtigt) links vom Scheitelpunkt $A^1$ und $B^2$ in entsprechender Weise das nächstliegende ausgeprägte Minimum rechts vom Scheitelpunkt $A^1$ und bezeichnet $C^1$ den Punkt, an dem eine vom Scheitelpunkt $A^1$ senkrecht zur $2\Theta$-Achse gezogene Gerade eine die Punkte $B^1$ und $B^2$ verbindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der $2\Theta$ Achse und der Intensitätsachse herangezogen werden.

Die Halbwertsbreite ist in dieser Schrift in entsprechender Weise die Länge des Geradenabschnitts, der sich zwischen den beiden Schnittpunkten $H^1$ und $H^2$ ergibt, wenn man in der Mitte des Geradenabschnitts $A^1C^1$ eine Parallele zur $2\Theta$-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen.

Eine beispielhafte Durchführung der Bestimmung von Halbwertsbreite und Intensität zeigt auch die Figur 6 in der DE-A 10046672.

Selbstredend können die erfindungsgemäß erhältlichen Multimetalloxidmassen M auch in mit feinteiligen, z.B. kolloidalen, Materialien, wie Siliciumdioxid, Titandioxid, Aluminiumoxid, Zirkonoxid, Nióboxid, verdünnter Form als katalytische Aktivmassen eingesetzt werden.

Das Verdünnungsmassenverhältnis kann dabei bis zu 9 (Verdünner): 1 (Aktivmasse) betragen. D.h., mögliche Verdünnungsmassenverhältnisse betragen z.B. 6 (Verdünner) : 1 (Aktivmasse) und 3 (Verdünner): 1 (Aktivmasse). Die Einarbeitung der Verdünner kann vor und/oder nach der Calcination im Rahmen der Herstellung der erfindungsgemäß zu waschenden Multimetalloxidmasse M, in der Regel sogar vor der Trocknung erfolgen.

Erfolgt die Einarbeitung vor der Trocknung bzw. vor der Calcination, muß der Verdünner so gewählt werden, dass er im fluiden Medium bzw. bei der Calcination im wesentlichen erhalten bleibt. Dies ist z.B. im Fall von bei entsprechend hohen Temperaturen gebrannten Oxiden in der Regel gegeben.

Die erfindungsgemäß erhältlichen Multimetalloxidmassen M eignen sich, wie bereits gesagt, als solche oder in wie eben beschrieben verdünnter Form als Aktivmassen für heterogen katalysierte partielle Gasphasenoxidationen (einschließlich Oxidehydrierungen) und/oder -ammoxidationen von gesättigten und/oder ungesättigten Kohlenwasserstoffen.

Solche gesättigten und/oder ungesättigten Kohlenwasserstoffe sind insbesondere Ethan, Ethylen, Propan, Propylen, n-Butan, iso-Butan und Butene wie z.B. 2-Buten und iso-Buten. Zielprodukte sind dabei vor allem Acrolein, Acrylsäure, Methacrolein, Methacrylsäure, Acrylnitril und Methacrylnitril. Sie eignen sich aber auch für die heterogen katalysierte partielle Gasphasenoxidation und/oder -ammoxidation von Verbindungen wie Acrolein und Methacrolein.

Aber auch Ethylen, Propylen und Essigsäure können Zielprodukt sein.

Unter einer vollständigen Oxidation des Kohlenwasserstoffs wird in dieser Schrift verstanden, dass der im Kohlenwasserstoff insgesamt enthaltene Kohlenstoff in Oxide des Kohlenstoffs ($CO$, $CO_2$) umgewandelt wird.

Alle davon verschiedenen Umsetzungen des Kohlenwasserstoffs unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die partielle Ammoxidation.

Bevorzugt eignen sich die in dieser Schrift niedergelegten erfindungsgemäß erhältlichen Multimetalloxidmassen M als katalytische Aktivmassen für die Umsetzung von Propan zu Acrolein und/oder Acrylsäure, von Propan zu Acrylsäure und/oder Acrylnitril, von Propylen zu Acrolein und/oder Acrylsäure, von Propylen zu Acrylnitril, von iso-Butan zu Methacrolein und/oder Methacrylsäure, von iso-Butan zu Methacrylsäure und/oder Methacrylnitril, von Ethan zu Ethylen, von Ethan zu Essigsäure und von Ethylen zu Essigsäure.

Die Durchführung solcher partiellen Oxidationen und/oder Ammoxidationen (durch in an sich bekannter Weise zu steuernde Wahl des Gehaltes an Ammoniak im Reaktionsgasgemisch kann die Reaktion im wesentlichen ausschließlich als partielle Oxidation, oder ausschließlich als partielle Ammoxidation, oder als Überlagerung beider Reaktionen gestaltet werden; vgl. z.B. WO 98/22421) ist an sich bekannt und kann in völlig entsprechender Weise durchgeführt werden.

Wird als Kohlenwasserstoff Roh-Propan oder Roh-Propylen eingesetzt, ist dieses bevorzugt wie in der DE-A 10246119 bzw. DE-A 10118814 bzw. PCT/EP/02/04073 beschrieben zusammengesetzt. Ebenso wird bevorzugt wie dort beschrieben verfahren.

Eine mit erfindungsgemäß erhältlichen Multimetalloxid-M-aktivmasse-Katalysatoren durchzuführende partielle Oxidation von Propan zu Acrylsäure kann z.B. wie in der EP-A 608838, der WO 0029106, der JP-A 10-36311, der EP-A 1193240 und der EP-A 1192987 beschrieben durchgeführt werden.

Als Quelle für den benötigten molekularen Sauerstoff kann z.B. Luft, mit Sauerstoff angreicherte oder an Sauerstoff entreicherte Luft oder reiner Sauerstoff verwendet werden.

Ein solches Verfahren ist auch dann vorteilhaft, wenn das Reaktionsgasausgangsgemisch kein Edelgas, insbesondere kein Helium, als inertes Verdünnungsgas enthält. Im übrigen kann das Reaktionsgasausgangsgemisch neben Propan und molekularem Sauerstoff selbstredend inerte Verdünnungsgase wie z.B. $N_2$, $CO$ und $CO_2$ umfassen. Wasserdampf als Reaktionsgasgemischbestandteil ist erfindungsgemäß vorteilhaft.

D.h., das Reaktionsgasausgangsgemisch, mit dem die erfindugsgemäß erhältliche Multimetalloxidaktivmasse M bei Reaktionstemperaturen von z.B. 200 bis 550°C oder von 230 bis 480°C bzw. 300 bis 440°C und Drücken von 1 bis 10 bar, bzw. 2 bis 5 bar zu belasten ist, kann z.B. nachfolgende Zusammensetzung aufweisen:

> 1 bis 15, vorzugsweise 1 bis 7 Vol.-% Propan,
> 44 bis 99 Vol.-% Luft und
> 0 bis 55 Vol.-% Wasserdampf.

Bevorzugt sind Wasserdampf enthaltende Reaktionsgasausgangsgemische.

Als andere mögliche Zusammensetzungen des Reaktionsgasausgangsgemisches kommen in Betracht:

> 70 bis 95 Vol.-% Propan,

5 bis 30 Vol.-% molekularer Sauerstoff und

0 bis 25 Vol.-% Wasserdampf.

Selbstredend wird bei einem solchen Verfahren ein Produktgasgemisch erhalten, das nicht ausschließlich aus Acrylsäure besteht. Vielmehr enthält das Produktgasgemisch neben nicht umgesetztem Propan Nebenkomponenten wie Propen, Acrolein, $CO_2$, CO, $H_2O$, Essigsäure, Propionsäure etc., von denen die Acrylsäure abgetrennt werden muß.

Dies kann so erfolgen, wie es von der heterogen katalysierten Gasphasenoxidation von Propen zu Acrylsäure bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierende Kondensation erfolgen, wie es z.B. in der DE-A 19 924 532 beschrieben ist.

Das dabei resultierende wäßrige Acrylsäurekondensat kann dann z.B. durch fraktionierende Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan, welches vorzugsweise in die Gasphasenoxidation rückgeführt wird. Es kann dazu aus dem Restgasgemisch z.B. durch fraktionierende Druckrektifikation teil- oder vollabgetrennt und anschließend in die Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propan bevorzugt zu absorbieren vermag.

Durch nachfolgende Desorption und/oder Strippung mit Luft kann das absorbierte Propan wieder freigesetzt und in das erfindungsgemäße Verfahren rückgeführt werden. Auf diese Weise sind wirtschaftliche Gesamtpropanumsätze erzielbar. Als Nebenkomponente gebildetes Propen wird dabei, ebenso wie bei anderen Abtrennverfahren, in der Regel vom Propan nicht oder nicht vollständig abgetrennt und mit diesem im Kreis geführt. Dies gilt so auch im Fall von anderen homologen gesättigten und olefinischen Kohlenwasserstoffen. Vor allem gilt es ganz generell für mit erfindungsgemäß erhältlichen Multimetalloxidmassen M heterogen katalysierte partielle Oxidationen und/oder -ammoxidationen von gesättigten Kohlenwasserstoffen.

Dabei macht es sich vorteilhaft bemerkbar, daß die erfindungsgemäß erhältlichen Multimetalloxidmassen M auch die partielle Oxidation und/oder -ammoxidation des homologen olefinischen Kohlenwasserstoffs zum selben Zielprodukt heterogen zu katalysieren vermögen.

So kann mit den erfindungsgemäß erhältlichen Multimetalloxidmassen M als Aktivmassen Acrylsäure durch heterogen katalysierte partielle Gasphasenoxidation von Propen mit molekularem Sauerstoff wie in der DE-A 10118814 bzw. PCT/EP/02/04073 oder der JP-A 7-53448 beschrieben hergestellt werden.

D.h., eine einzige Reaktionszone A ist für die Durchführung des erfindungsgemäßen Verfahrens ausreichend. In dieser Reaktionszone befinden sich als katalytisch aktive Massen ausschließlich erfindungsgemäß erhältliche Multimetalloxidmasse-M-Katalysatoren.

Dies ist ungewöhnlich, verläuft die heterogen katalysierte Gasphasenoxidation von Propen zu Acrylsäure doch ganz allgemein in zwei zeitlich aufeinanderfolgenden Schritten. Im ersten Schritt wird üblicherweise Propen im wesentlichen zu Acrolein oxidiert und im zweiten Schritt wird üblicherweise im ersten Schritt gebildetes Acrolein zu Acrylsäure oxidiert.

Konventionelle Verfahren der heterogen katalysierten Gasphasen-oxidation von Propen zu Acrylsäure setzen daher üblicherweise für jeden der beiden vorgenannten Oxidationsschritte einen speziellen, auf den Oxidationsschritt maßgeschneiderten, Katalysatortyp ein.

D.h., die konventionellen Verfahren der heterogen katalysierten Gasphasenoxidation von Propen zu Acrylsäure arbeiten im Unterschied zum erfindungsgemäßen Verfahren mit zwei Reaktionszonen.

Selbstredend kann sich beim mit erfindungsgemäß erhältlichen Multimetalloxidmassen M katalysierten Verfahren der Propenpartialoxidation in der einen Reaktionszone A nur ein oder aber auch mehr als ein erfindungsgemäß erhältlicher Multimetalloxidmasse-M-Katalysator befinden. Natürlich können diese erfindungsgemäß erhältlichen Katalysatoren mit Inertmaterial verdünnt sein, wie es in dieser Schrift beispielsweise auch als Trägermaterial empfohlen wurde.

Längs der einen Reaktionszone A kann beim mit erfindungsgemäß erhältlichen Multimetalloxidmassen M katalysierten Verfahren der Propenpartialoxidation nur eine oder aber auch eine sich längs der Reaktionszone A ändernde Temperatur eines Wärmeträgers zur Temperierung der Reaktionszone A herrschen. Diese Temperaturänderung kann zunehmend oder abnehmend sein.

Wird ein solches Verfahren der Propenpartialoxidation als Festbettoxidation ausgeführt, erfolgt die Durchführung in zweckmäßiger Weise in einem Rohrbündelreaktor, dessen Kontaktrohre mit dem Katalysator beschickt sind.

Um die Kontaktrohre wird im Normalfall als Wärmeträger eine Flüssigkeit, in der Regel ein Salzbad geführt.

Mehrere Temperaturzonen längs der Reaktionszone A können dann in einfacher Weise dadurch realisiert werden, daß längs der Kontaktrohre abschnittsweise mehr als ein Salzbad um die Kontaktrohre geführt wird.

Das Reaktionsgasgemisch wird in den Kontaktrohren über den Reaktor betrachtet entweder im Gleichstrom oder im Gegenstrom zum Salzbad geführt. Das Salzbad selbst kann relativ zu den Kontaktrohren eine reine Parallelströmung ausführen. Selbstverständlich kann dieser aber auch eine Querströmung überlagert sein. Insgesamt kann das Salzbad um die Kontaktrohre auch eine mäanderförmige Strömung ausführen, die nur über den Reaktor betrachtet im Gleich- oder im Gegenstrom zum Reaktionsgasgemisch geführt ist.

Die Reaktionstemperatur kann beim erfindungsgemäßen Verfahren der Propenpartialoxidation längs der gesamten Reaktionszone A 200° bis 500°C betragen. Üblicherweise wird sie 250 bis 450°C betragen. Bevorzugt wird die Reaktionstemperatur 330 bis 420°C, besonders bevorzugt 350 bis 400°C betragen.

Der Arbeitsdruck kann beim mit erfindungsgemäß erhältlichen Multimetalloxid-M-katalysatoren katalysierten Verfahren der Propenpartialoxidation sowohl 1 bar, weniger als 1 bar oder mehr als 1 bar betragen. Erfindungsgemäß typische Arbeitsdrücke sind 1,5 bis 10 bar, häufig 1,5 bis 5 bar.

An das für dieses Verfahren der Propenpartialoxidation zu verwendende Propen werden keine besonders hohen Ansprüche im Bezug auf seine Reinheit gestellt.

Als Propen kann für ein solches Verfahren, wie bereits gesagt und wie für alle ein- oder zweistufigen Verfahren der heterogen katalysierten Gasphasenoxidation von Propen zu Acrolein und/oder Acrylsäure ganz generell, z.B. Propen (auch Roh-Propen genannt) der nachfolgenden beiden Spezifikationen völlig problemlos verwendet werden:

a) Polymer grade Propylen:

| | |
|---|---|
| $\geq$ 99,6 gew.-% | Propen, |
| $\leq$ 0,4 gew.-% | Propan, |
| $\leq$ 300 gew.ppm | Ethan und/oder Methan, |
| $\leq$ 5 gew.ppm | $C_4$-Kohlenwasserstoffe, |
| $\leq$ 1 gew.ppm | Acetylen, |
| $\leq$ 7 gew.ppm | Ethylen, |
| $\leq$ 5 gew.ppm | Wasser, |
| $\leq$ 2 gew.ppm | $O_2$, |
| $\leq$ 2 gew.ppm | Schwefel enthaltende Verbindungen (berechnet als Schwefel), |
| $\leq$ 1 gew.ppm | Chlor enthaltende Verbindungen (berechnet als Chlor), |
| $\leq$ 5 gew.ppm | $CO_2$, |
| $\leq$ 5 gew.ppm | CO, |
| $\leq$ 10 gew.ppm | Cyclopropan, |
| $\leq$ 5 gew.ppm | Propadien und/oder Propin, |
| $\leq$ 10 gew.ppm | $C_{\geq 5}$-Kohlenwasserstoffe und |
| $\leq$ 10 gew.ppm | Carbonylgruppen enthaltende Verbindungen (berechnet als $Ni(CO)_4$). |

b) Chemical grade Propylen:

| | |
|---|---|
| $\geq$ 94 gew.-% | Propen, |
| $\leq$ 6 gew.-% | Propan, |
| $\leq$ 0,2 gew.-% | Methan und/oder Ethan, |
| $\leq$ gew.ppm | Ethylen, |
| $\leq$ 1 gew.ppm | Acetylen, |
| $\leq$ 20 gew.ppm | Propadien und/oder Propin, |
| $\leq$ 100 gew.ppm | Cyclopropan, |

(fortgesetzt)

| | |
|---|---|
| $\leq 50$ gew.ppm | Buten, |
| $\leq 50$ gew.ppm | Butadien, |
| $\leq 200$ gew.ppm | $C_4$-Kohlenwasserstoffe, |
| $\leq 10$ gew.ppm | $C_{\geq 5}$-Kohlenwasserstoffe, |
| $\leq 2$ gew.ppm | Schwefel enthaltende Verbindungen (berechnet als Schwefel), |
| $\leq 0,1$ gew.ppm | Sulfide (berechnet als $H_2S$), |
| $\leq 1$ gew.ppm | Chlor enthaltende Verbindungen (berechnet als Chlor), |
| $\leq 0,1$ gew.ppm | Chloride (berechnet als $Cl^{\ominus}$) und |
| $\leq 30$ gew.ppm | Wasser. |

Selbstverständlich können alle vorstehend genannten möglichen Begleiter des Propens jeweils aber auch in der zwei- bis zehnfachen der genannten individuellen Menge im Roh-Propen enthalten sein, ohne die Verwendbarkeit des Roh-Propens für das beschriebene Verfahren bzw. für die bekannten Verfahren der ein- oder zweistufigen heterogen katalysierten Gasphasenoxidation von Propen zu Acrolein und/oder Acrylsäure ganz generell, zu beeinträchtigen.

Dies gilt insbesondere dann, wenn es sich wie bei den gesättigten Kohlenwasserstoffen, dem Wasserdampf, den Kohlenoxiden oder dem molekularen Sauerstoff sowieso um Verbindungen handelt, die entweder als inerte Verdünnungsgase oder als Reaktionspartner in erhöhten Mengen bei den vorgenannten Verfahren am Reaktionsgeschehen teilnehmen. Normalerweise wird das Roh-Propen als solches mit Kreisgas, Luft und/oder molekularem Sauerstoff und/oder verdünnter Luft und/oder Inertgas vermischt für das beschriebene Verfahren und alle sonstigen Verfahren der heterogen katalysierten Gasphasenoxidation von Propen zu Acrolein und/oder Acrylsäure eingesetzt.

Als Propenquelle kommt für das beschriebene Verfahren aber auch Propen in Betracht, das bis zu 40 % seines Gewichts Propan enthält. Dabei kann dieses Propen zusätzlich noch von anderen, das mit erfindungsgemäß erhältlichen Multimetalloxidmassen-M-katalysatoren katalysierte Verfahren im wesentlichen nicht störenden, Begleitkomponenten begleitet werden.

Als Sauerstoffquelle kann für dieses Verfahren der Propenpartialoxidation sowohl reiner Sauerstoff als auch Luft oder mit Sauerstoff angereicherte bzw. abgereicherte Luft verwendet werden.

Neben molekularem Sauerstoff und Propen enthält ein für ein solches Verfahren zu verwendendes Reaktionsgasausgangsgemisch üblicherweise noch wenigstens ein Verdünnungsgas. Als solches kommen Stickstoff, Kohlenoxide, Edelgase und niedere Kohlenwasserstoffe wie Methan, Ethan und Propan in Betracht (höhere, z.B. $C_4$-,Kohlenwasserstoffe sollten gemieden werden). Häufig wird auch Wasserdampf als Verdünnungsgas verwendet. Vielfach bilden Mischungen aus vorgenannten Gasen das Verdünnungsgas für das erfindungsgemäße Verfahren der partiellen Propenoxidation.

Vorteilhaft erfolgt die beschriebene heterogen katalysierte Partialoxidation des Propens im Beisein von Propan.

In typischer Weise ist das Reaktionsgasausgangsgemisch für ein Propenpartialoxidationsverfahren wie folgt zusammengesetzt (molare Verhältnisse):

Propen : Sauerstoff : $H_2O$ : sonstige Verdünnungsgase = 1 : (0,1 - 10) : (0 - 70) : (0 - 20).

Vorzugsweise beträgt das vorgenannte Verhältnis 1 : (1-5) : (1 - 40) : (0 - 10).

Wird Propan als Verdünnungsgas verwendet, kann dieses, wie be-schrieben, beim beschriebenen Verfahren vorteilhaft teilweise ebenfalls zu Acrylsäure oxidiert werden.

Bevorzugt enthält das Reaktionsgasausgangsgemisch molekularen Stickstoff, CO, $CO_2$, Wasserdampf und Propan als Verdünnungsgas.

Das molare Verhältnis von Propan : Propen kann beim beschriebenen Verfahren folgende Werte annehmen: 0 bis 15, häufig 0 bis 10, vielfach 0 bis 5, zweckmäßig 0,01 bis 3.

Die Belastung der Katalysatorbeschickung mit Propen kann beim beschriebenen Verfahren der partiellen Propenoxidation z.B. 40 bis 250 Nl/l·h betragen. Die Belastung mit Reaktionsgasausgangs-gemisch liegt häufig im Bereich von 500 bis 15000 Nl/l·h, vielfach im Bereich 600 bis 10000 Nl/l·h, häufig 700 bis 5000 Nl/l·h.

Selbstredend wird beim beschriebenen Verfahren der Propenpartialoxidation zu Acrylsäure ein Produktgasgemisch erhalten, das nicht ausschließlich aus Acrylsäure besteht. Vielmehr enthält das Produktgasgemisch neben nicht umgesetztem Propen Nebenkomponenten wie Propan, Acrolein, $CO_2$, CO, $H_2O$, Essigsäure, Propionsäure etc., von denen die Acrylsäure abgetrennt werden muß.

Dies kann so erfolgen, wie es von der heterogen katalysierten zweistufigen (in zwei Reaktionszonen durchgeführten) Gasphasenoxidation von Propen zu Acrylsäure allgemein bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierende Kondensation erfolgen, wie es z.B. in der DE-A 19 924 532 beschrieben ist.

Das dabei resultierende wäßrige Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propen (und gegebenenfalls Propan). Dieses kann aus dem Restgasgemisch z.B. durch fraktionierte Druckrektifikation abgetrennt und anschließend in die beschriebene Gasphasenpartialoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propen (und gegebenenfalls Propan) bevorzugt zu absorbieren vermag.

Durch nachfolgende Desorption und/oder Strippung mit Luft kann das absorbierte Propen (und gegebenenfalls Propan) wieder freigesetzt und in das erfindungsgemäße Verfahren rückgeführt werden. Auf diese Weise sind wirtschaftliche Gesamtpropenumsätze erzielbar. Wird Propen im Beisein von Propan partialoxidiert, werden Propen und Propan bevorzugt gemeinsam abgetrennt und rückgeführt.

In völlig entsprechender Weise lassen sich die erfindungsgemäß erhältlichen Multimetalloxide M als Katalysatoren für die Partialoxidation von iso-Butan und/oder iso-Buten zu Methacrylsäure einsetzen.

Ihre Verwendung für die Ammoxidation von Propan und/oder Propen kann z.B. wie in der EP-A 529853, der DE-A 2351151, der JP-A 6-166668 und der JP-A 7-232071 beschrieben erfolgen.

Ihre Verwendung für die Ammoxidation von n-Butan und/oder n-Buten kann wie in der JP-A 6-211767 beschrieben erfolgen.

Ihre Verwendung für die Oxidehydrierung von Ethan zu Ethylen, bzw. die Weiterreaktion zu Essigsäure, kann wie in der US-A 4250346 oder wie in der EP-B 261264 beschrieben erfolgen.

Die erfindungsgemäß erhältlichen Multimetalloxidmassen M können aber auch in andere Multimetalloxidmassen integriert werden (z.B. ihre feinteiligen Massen vermengen, gegebenenfalls verpressen und calcinieren, oder als Schlämmen (vorzugsweise wässrig) vermengen, trocknen und calcinieren (z.B. wie es die EP-A 529853 für erfindungsgemäß zu waschende Multimetalloxidmassen M mit d = 0 beschreibt)). Bevorzugt wird wieder unter Inertgas calciniert.

Die dabei resultierenden Multimetalloxidmassen (nachfolgend Gesamtmassen genannt) enthalten bevorzugt $\geq 50$ Gew.-%, besonders bevorzugt $\geq 75$ Gew.-%, und ganz besonders bevorzugt $\geq 90$ Gew.-% bzw. $\geq 95$ Gew.-% an erfindungsgemäß erhältlichen Multimetalloxidmassen M und sind für die in dieser Schrift besprochenen Partialoxidationen und/oder -ammoxidationen ebenfalls geeignet.

Bevorzugt enthalten auch die Gesamtmassen bei $2\Theta = 50{,}0 \pm 3{,}0°$ keine Beugungsreflex-Scheitelpunktlage.

Enthält die Gesamtmasse bei $2\Theta = 50{,}0 \pm 0{,}3°$ eine Beugungsrelfex-Scheitelpunktlage, ist es günstig, wenn der Gewichtsanteil der erfindungsgemäß erhältlichen Multimetalloxidmassen M $\geq 80$ Gew.-%, oder $\geq 90$ Gew.-%, oder $\geq 95$ Gew.-% beträgt.

Die geometrische Formgebung erfolgt bei den Gesamtmassen in zweckmäßiger Weise wie für die erfindungsgemäß erhältlichen Multimetalloxidmassen M beschrieben.

Die Vorteilhaftigkeit der erfindungsgemäß erhältlichen Multimetalloxidmassen M basiert auf ihrer hervorragenden Zielproduktselektivität, und zwar sowohl bezüglich der in der Schrift genannten Partialoxidationen als auch Partialammoxidationen.

Zum Zweck der heterogen katalysierten partiellen Gasphasenoxidation von Propan zu Acrylsäure werden die erfindungsgemäß erhältlichen Multimetalloxidmassen M und diese enthaltende Multi-metalloxidmassen bzw. Katalysatoren bevorzugt wie in der DE-A 10122027 beschrieben in Betrieb genommen.

Beispiele

A) Herstellung von Multimetalloxidmassen-Schalenkatalysatoren

**Vergleichsbeispiel** (Nicht erfindungsgemäß Herstellung eines Multimetalloxidkatalysators mit einer Aktivmasse $Mo_{1,0}V_{0,28}Te_{0,11}Nb_{0,16}O_x$).

100 g Ammoniumheptamolybdat (81,5 Gew.-% $MoO_3$, Fa. Starck/Goslar) wurden zusammen mit 9,4 g Tellur-

säure (99 Gew.-% $H_6TeO_6$, Fa. Aldrich) bei 50°C in der genannten Reihenfolge in 423 ml Wasser gelöst, wobei eine Lösung 1 erhalten wurde.

In einem zweiten Behälter wurden ebenfalls bei 50°C 35,83 g Ammoniumnioboxalat (21,0 Gew.-% Nb, Fa. Starck/Goslar) zusammen mit 9,4 g der vorgenannten Tellursäure in der genannten Reihenfolge in 423 ml Wasser gelöst, wobei eine Lösung 2 erhalten wurde. Eine dritte Lösung wurde dadurch hergestellt, daß bei 50°C 41,1 g Vanadium-(IV)-oxysulfat (20,1 Gew.-% V, Fa. Aldrich) in 500 ml Wasser gelöst wurden, wobei eine Lösung 3 erhalten wurde. Unter Rühren wurde dann unter Beibehalt der 50°C die Lösung 2 innerhalb von 0,5 min. in die Lösung 1 eingerührt. In das dabei resultierende Gemisch wurde ebenfalls unter Beibehalt der 50°C innerhalb von 1,5 min. Lösung 3 eingerührt. Die dabei resultierende wäßrige Suspension wurde anschließend in einen Stahlautoklaven mit einem Innenvolumen von 2300 ml gefüllt und ohne Rühren im geschlossenen Autoklaven auf 175°C aufgeheizt (innerhalb von 60 min.). Nach einer ungerührten Verweilzeit von 48 h bei 175°C im Autoklaven wird der Autoklav durch sich selbst überlassen auf 25°C abgekühlt, geöffnet und der in der flüssigen Phase enthaltene Feststoff aus der Lösung abfiltriert, mit 50 ml Wasser der Temperatur 25°C gewaschen und bei einer Temperatur von 40°C während 12 h getrocknet.

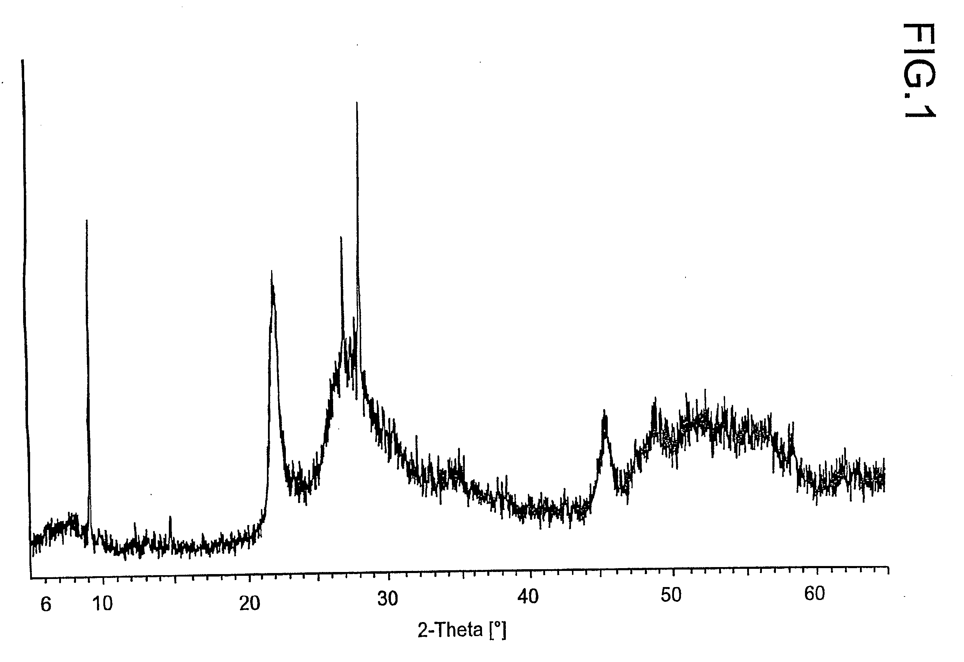

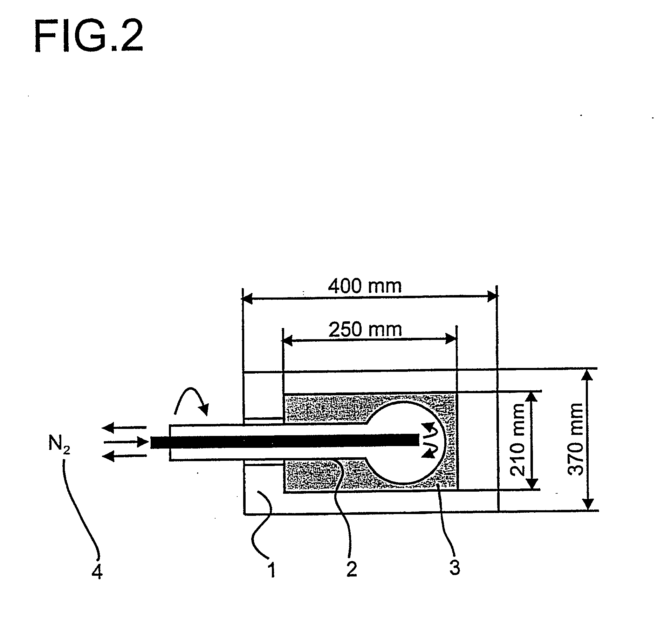

100 g des getrockneten Feststoffs, dessen Röntgendiffraktogramm Fig. 1 zeigt, wurden in einem Drehkugelofen gemäß Figur 2 (Quarzglaskugel mit 1 Liter Innenvolumen; 1 = Ofengehäuse, 2 = Drehkolben, 3 = beheizter Raum, 4 = Stickstoff-/Luftstrom) unter einem Luftstrom von 50 Nl/h innerhalb von 25 min. zunächst linear von 25°C auf 250°C aufgeheizt und diese Temperatur und der Luftstrom anschließend für 1 h aufrechterhalten. Unmittelbar daran anschließend wurde der Luftstrom durch einen Stickstoffstrom von 50 Nl/h ersetzt und innerhalb von 35 min. linear von 250°C auf 600°C aufgeheizt. Diese Temperatur und der Stickstoffstrom wurden dann während 2 h aufrechterhalten. Abschließend wurde unter Aufrechterhaltung des Stickstoffstromes der gesamte Drehkugelofen auf 25°C abgekühlt.

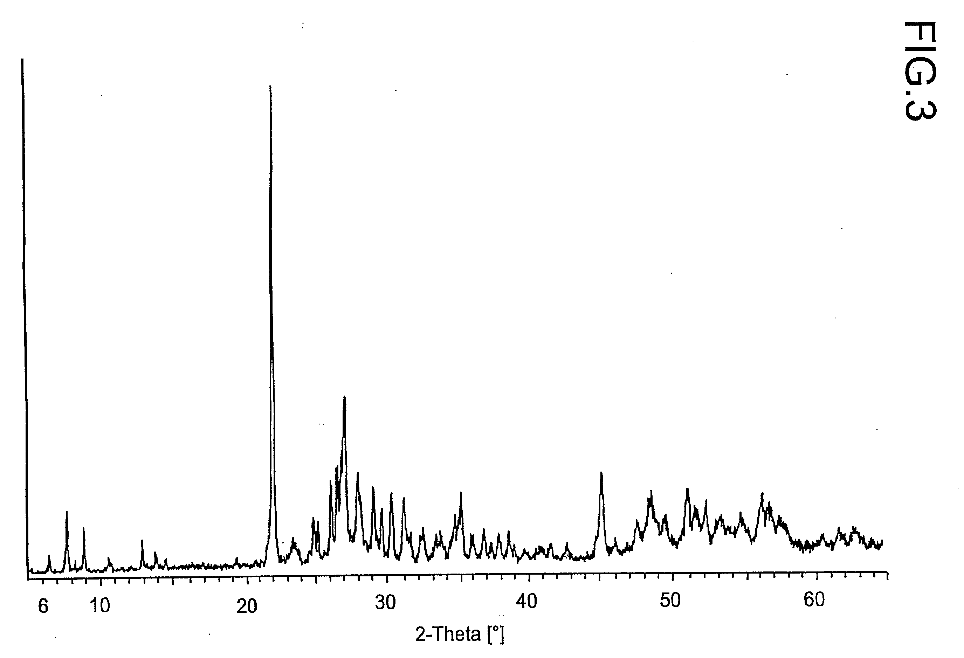

Es wurde ein schwarzes Pulver der Zusammensetzung $Mo_{1,0}V_{0,28}Te_{0,11}Nb_{0,16}O_x$ erhalten. Das zugehörige Röntgendiffraktogramm zeigt die Figur 3.

Das Aktivmassenpulver wurde anschließend in einer Retsch-Mühle (Zentrifugalmühle, Typ ZM 100, Fa. Retsch, DE) gemahlen (Korngröße $\leq$ 0,12 mm).

38 g des nach Mahlung vorliegenden Pulvers wurde auf 150 g kugelförmige Trägerkörper mit einem Durchmesser von 2,2 bis 3,2 mm ($R_z$ = 45 $\mu$m, Trägermaterial = Steatit der Fa. Ceramtec, DE, Porengesamtvolumen des Trägers $\leq$ 1 Vol.-% bezogen auf das Trägergesamtvolumen) aufgebracht. Dazu wurde der Träger in eine Dragiertrommel mit 2 l Innenvolumen (Neigungswinkel der Trommelmittelachse gegen die Horizontale = 30°) vorgelegt. Die Trommel wurde mit 25 Umdrehungen je Minute in Rotation versetzt. Über eine mit 300 Nl/h Druckluft betriebene Zerstäuberdüse wurden über 60 min. hinweg ca. 25 ml eines Gemisches aus Glycerin und Wasser (Gewichtsverhältnis Glycerin: Wasser = 1:3) auf den Träger gesprüht. Die Düse war dabei derart installiert, daß der Sprühkegel die in der Trommel durch Mitnahmebleche an den obersten Punkt der geneigten Trommel beförderten Trägerkörper in der oberen Hälfte der Abrollstrecke benetzte. Das feinteilige Aktivmassenpulver wurde über eine Pulverschnecke in die Trommel eingetragen, wobei der Punkt der Pulverzugabe innerhalb der Abrollstrecke oder unterhalb des Sprühkegels lag. Durch die periodische Wiederholung von Benetzung und Pulveraufdosierung wurde der grundbeschichtete Trägerkörper in der darauffolgenden Periode selbst zum Trägerkörper.

Nach Abschluß der Beschichtung wurde der beschichtete Trägerkörper unter Luft während 16 h bei 150°C im Muffelofen getrocknet. Es resultierte ein Schalenkatalysator VB1 mit 20 Gew.-% Aktivmassenanteil.

**Beispiel** (nicht erfindungsgemäß Herstellung eines Multimetalloxidkatalysators mit einer Aktivmasse $Mo_{1,0}V_{0,22}Te_{0,09}Nb_{0,17}O_x$)

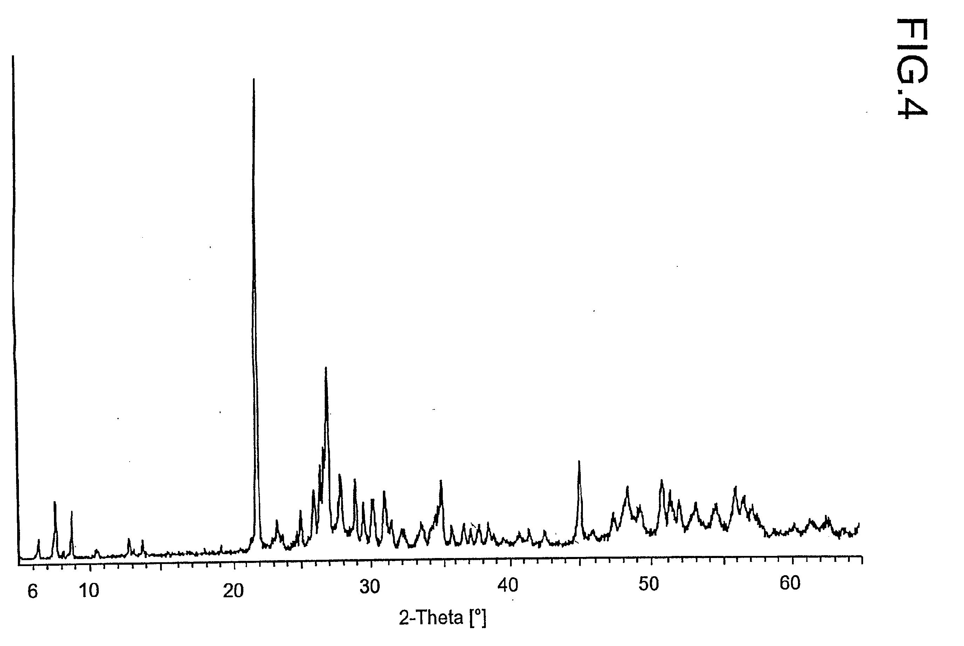

100 g an schwarzem Aktivmassenpulver $Mo_{1,0}V_{0,28}Te_{0,11}Nb_{0,16}O_x$ gemäß des Vergleichsbeispiels wurden in 1000 ml einer 10 gew.-%igen wäßrigen $HNO_3$-Lösung während 7 h bei 70°C unter Rückfluß gerührt. Der dabei verbliebene Feststoff wurde aus der resultierenden Aufschlämmung abfiltriert und mit Wasser nitratfrei gewaschen. Der dabei verbliebene Filterkuchen wurde 12 h unter Luft bei 110°C in einem Muffelofen getrocknet. Die resultierende trockene Aktivmasse hatte die Zusammensetzung $Mo_{1,0}V_{0,22}Te_{0,09}Nb_{0,17}O_x$. Das zugehörige Röntgendiffraktogramm zeigt die Figur 4.

Sie wurde in gleicher Weise wie im Vergleichsbeispiel gemahlen und auf denselben Träger aufgebracht, so dass ein Schalenkatalysator B1 mit ebenfalls 20 Gew.-% Aktivmassenanteil resultierte.

B) Testung der in A) hergestellten Multimetalloxidmassen-Schalenkatalysatoren

Mit jeweils 35,0 g des jeweiligen Schalenkatalysators aus A) wurde ein aus Stahl gefertigter Rohrreaktor (Innendurchmesser: 8,5 mm, Länge: 140 cm, Wanddicke: 2,5 cm) beschickt (Katalysatorschüttlänge in allen Fällen ca. 53 cm). Vor der Katalysatorschüttung wurde eine Vorschüttung von 30 cm Steatitkugeln (Durchmesser: 2,2 bis 3,2 mm, Hersteller: Fa. Ceramtec) und nach der Katalysatorschüttung auf der Restlänge des Rohrreaktors eine Nachschüttung derselben Steatitkugeln angebracht.

Mittels elektrisch beheizter Heizmatten wurde von außen die Außentemperatur des beschickten Reaktionsrohres auf der gesamten Länge auf 350°C eingestellt.

Dann wurde das Reaktionsrohr mit einem Reaktionsgasausgangsgemisch der molaren Zusammensetzung Propan: Luft: $H_2O$ = 1:15:14 beschickt (die Eintrittsseite war auf der Seite der Nachschüttung). Die Verweilzeit (bezogen auf das Katalysatorschüttungsvolumen) wurde auf 2,4 sec. eingestellt. Der Eingangsdruck betrug 2 bar absolut.

Die Reaktionsrohrbeschickung wurde zunächst jeweils bei der vorgenannten Außentemperatur des beschickten Reaktionsrohres über einen Zeitraum von 24 h eingefahren, bevor diese Außentemperatur auf die in der nachfolgenden Tabelle aufgeführte jeweilige Reaktionstemperatur erhöht wurde.

Die nachfolgende Tabelle zeigt in Abhängigkeit vom verwendeten Schalenkatalysator den bei dieser Außentemperatur T (°C) auf einmaligen Reaktionsrohrdurchgang bezogenen resultierenden Umsatz des Propans ($U_{PAN}$), sowie die dabei resultierende Selektivität der Acrylsäurebildung ($S_{ACS}$ (mol-%)) und die Selektivität der Nebenproduktbildung an Propen ($S_{PEN}$ (mol-%)). Zusätzlich zeigt die Tabelle das Intensitätsverhältnis R der auf dem Schalenkatalysator befindlichen Aktivmasse.

Tabelle

| Beispiel | R | T [°C] | $U_{PAN}$ (mol-%) | $S_{ACS}$ (mol-%) | $S_{PEN}$ (mol-%) |
|---|---|---|---|---|---|
| VB 1 | 0,66 | 390 | 15,42 | 49,23 | 19,55 |
| B1 | 0,75 | 390 | 30,73 | 67,63 | 9,21 |

**Patentansprüche**

1. Verfahren zur Herstellung einer Multimetalloxidmasse M der allgemeinen Stöchiometrie I

$$Mo_lV_aM^1{}_bM^2{}_cM^3{}_dO_n \qquad (I),$$

mit

M$^1$ = wenigstens eines der Elemente aus der Gruppe umfassend Te und Sb;
M$^2$ = wenigstens eines der Elemente aus der Gruppe umfassend Nb, Ti, W, Ta und Ce;
M$^3$ = wenigstens eines der Elemente aus der Gruppe umfassend Pb, Ni, Co, Bi, Pd, Ag, Pt, Cu, Au, Ga, Zn, Sn, In, Re, Ir, Sm, Sc, Y, Pr, Nd und Tb;
a = 0,01 bis 1,
b = > 0 bis 1,
c = > 0 bis 1,
d = > 0 bis 0,5 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2Θ) 22,2 $\pm$ 0,5° (h), 27,3 $\pm$ 0,5° (i) und 28,2 $\pm$ 0,5° (k) liegen, wobei

- der Beugungsreflex h innerhalb des Röntgendiffraktogramms der intensitätsstärkste ist, sowie eine Halbwertsbreite von höchstens 0,5° aufweist,
- die Intensität $P_i$ des Beugungsreflexes i und die Intensität $P_k$ des Beugungsreflexes k die Beziehung 0,65 $\leq$ R $\leq$ 0,85 erfüllen, in der R das durch die Formel

$$R = P_i \ / \ (P_i + P_k)$$

definierte Intensitätsverhältnis ist, und
- die Halbwertsbreite des Beugungsreflexes i und des Beugungsreflexes k jeweils $\leq$ 1° beträgt,

aber keinen Beugungsreflex mit der Scheitelpunktlage 2Θ = 50,0 $\pm$ 0,3° aufweist,
bei dem man zunächst eine solche Multimetalloxidmasse M mit der Maßgabe herstellt, daß im Verlauf der Herstellung dieser Multimetalloxidmasse M keine Vorläuferroultimetalloxidmasse dieser Multimetalloxidmasse M mit einer Flüssigkeit aus der Gruppe umfassend die Gruppenmitglieder organische Säuren, anorganische Säuren, Lösungen organischer Säuren, Lösungen anorganischer Säuren und Mischungen von vorgenannten Gruppenmitgliedern gewaschen wird, und das **dadurch gekennzeichnet ist, daß** die zunächst so hergestellte Multimetalloxidmasse M mit einer Flüssigkeit aus der Gruppe umfassend die Gruppenmitglieder organische Säuren, anorganische Säuren, Lösungen organischer Säuren, Lösungen anorganischer Säuren und Mischungen von vorgenannten Gruppenmitgliedern gewaschen wird.

2. Verfahren nach Anspruch 1, **dadurch gekennzeichnet, daß** die Flüssigkeit mit der gewaschen wird, eine wässrige Salpetersäurelösung ist.

3. Verfahren nach Anspruch 1, **dadurch gekennzeichnet, daß** das Röntgendiffraktogramm der zu waschenden Multimetalloxidmasse M neben den Beugungsreflexen h, i un k noch weitere Beugungsreflexe enthält, deren Scheitelpunkte bei den nachfolgenden Beugungswinkeln $2\Theta$ liegen:

    $9,0 \pm 0,4°$ (l),
    $6,7 \pm 0,4°$ (o), und
    $7,9 \pm 0,4°$ (p).

4. Verfahren nach Anspruch 3, **dadurch gekennzeichnet, daß** das Röntgendiffraktogramm der zu waschenden Multimetalloxidmasse M neben den Beugungsreflexen h, i, k, 1, o und p noch weitere Beugungsreflexe enthält, deren Scheitelpunkte bei den nachfolgenden Beugungswinkeln $2\Theta$ liegen:

    $45,2 \pm 0,4°$ (q),
    $29,2 \pm 0,4°$ (m), und
    $35,4 \pm 0,4°$ (n).

5. Verfahren nach Anspruch 4, **dadurch gekennzeichnet, daß** das Röntgendiffraktogramm der zu waschenden Multimetalloxidmasse M die Beugungsreflexe h, i, l, m, n, o, p und q in der gleichen Intensitätsskala mit den nachfolgenden Intensitäten aufweist:

    h: 100;
    i: 5 bis 95;
    l: 1 bis 30;
    m: 1 bis 40;
    n: 1 bis 40;
    o: 1 bis 30;
    p: 1 bis 30 und
    q: 5 bis 60.

6. Verfahren nach einem der Ansprüche 1 bis 5, **dadurch gekennzeichnet, daß** die stöchiometrischen Koeffizienten a, b, c und d der zu waschenden Multimetalloxidmasse M gleichzeitig im nachfolgenden Raster liegen:

    a = 0,05 bis 6;
    b = 0,01 bis 1;
    c = 0,01 bis 1; und
    d = 0,00005 bis 0,5.

7. Verfahren nach einem der Ansprüche 1 bis 6, **dadurch gekennzeichnet, daß** $M^1$ = Te.

8. Verfahren nach einem der Ansprüche 1 bis 7, **dadurch gekennzeichnet, daß** $M^2$ zu wenigstens 50 mol-% seiner Gesamtmenge Nb ist.

9. Verfahren nach einem der Ansprüche 6 bis 8, **dadurch gekennzeichnet, daß** $M^3$ wenigstens ein Element aus der Gruppe umfassend Ni, Co, Pd und Bi ist.

10. Verfahren nach einem der Ansprüche 1 bis 9, **dadurch gekennzeichnet, daß** die Herstellung der zu waschenden Multimetalloxidmasse M auf hydrothermalem Weg erfolgt.

**11.** Verfahren der heterogen katalysierten Gasphasenpartialoxidation und/oder -ammoxidation eines gesättigten und/oder ungesättigten Kohlenwasserstoffs, **dadurch gekennzeichnet, daß** als katalytisch aktive Masse das unmittelbare Verfahrensprodukt eines Verfahrens gemäß einem der Ansprüche 1 bis 10 verwendet wird.

**Claims**

**1.** A process for preparing a multimetal oxide material M of the stoichiometry I

$$Mo_1V_aM^1{}_bM^2cM^3{}_dO_n \qquad (I),$$

where

$M^1$ is at least one of the elements from the group consisting of Te and Sb;
$M^2$ is at least one of the elements from the group consisting of Nb, Ti, W, Ta and Ce;
$M^3$ is at least one of the elements from the group consisting of Pb, Ni, Co, Bi, Pd, Ag, Pt, Cu, Au, Ga, Zn, Sn, In, Re, Ir, Sm, Sc, Y, Pr, Nd and Tb;
a is from 0.01 to 1,
b is from > 0 to 1,
c is from > 0 to 1,
d is from > 0 to 0.5 and
n is a number which is determined by the valency and frequency of the elements other than oxygen in (I),

whose X-ray diffraction pattern has reflections h, i and k whose peaks are at the diffraction angles (2θ) 22.2 $\pm$ 0.5° (h), 27.3 $\pm$ 0.5° (i) and 28.2 $\pm$ 0.5° (k),

- the reflection h being the one with the strongest intensity within the X-ray diffraction pattern and having a full width at half height (FWHH) of not more than 0.5°,
- the intensity $P_i$ of the reflection i and the intensity $P_k$ of the reflection k satisfying the relationship 0.65 $\leq$ R $\leq$ 0.85, where R is the intensity ratio defined by the formula

$$R \ = \ P_i \ / \ (P_i \ + \ P_k)$$

and
- the FWHH of the reflection i and that of the reflection k being each $\leq$ 1°,

but has no reflection having the peak position 2θ = 50.0 $\pm$ 0.3°,
in which first such a multimetal oxide material M is prepared with the proviso that, in the course of the preparation of this multimetal oxide material M, no precursor multimetal oxide material of this multimetal oxide material M is washed with a liquid from the group consisting of organic acids, inorganic acids, solutions of organic acids, solutions of inorganic acids and mixtures of the abovementioned group members, and wherein the multimetal oxide material M initially prepared in this manner is washed with a liquid from the group consisting of organic acids, inorganic acids, solutions of organic acids, solutions of inorganic acids and mixtures of the abovementioned group members.

**2.** The process according to claim 1, wherein the liquid with which washing is effected is an aqueous nitric acid solution.

**3.** The process according to claim 1, wherein the X-ray diffraction pattern of the multimetal oxide material M to be washed contains, in addition to the reflections h, i and k, also further reflections whose peaks are at the following diffraction angles 2θ:

9.0 $\pm$ 0.4° (1),
6.7 $\pm$ 0.4° (o) and
7.9 $\pm$ 0.4° (p).

**4.** The process according to claim 3, wherein the X-ray diffraction pattern of the multimetal oxide material M to be washed contains, in addition to the reflections h, i, k, l, o and p, also further reflections whose peaks are at the

following diffraction angles $2\theta$:

45.2 $\pm$ 0.4° (q),
29.2 $\pm$ 0.4° (m) and
35.4 $\pm$ 0.4° (n).

5. The process according to claim 4, wherein the X-ray diffraction pattern of the multimetal oxide material M to be washed has the reflections h, i, l, m, n, o, p and q on the same intensity scale with the following intensities:

h = 100,
i = 5 to 95,
l = 1 to 30,
m = 1 to 40,
n = 1 to 40,
o = 1 to 30,
p = 1 to 30 and
q = 5 to 60.

6. The process according to any of claims 1 to 5, wherein the stoichiometric coefficients a, b, c and d of the multimetal oxide material M to be washed are simultaneously in the following ranges:

a = from 0.05 to 6;
b = from 0.01 to 1;
c = from 0.01 to 1; and
d = from 0.00005 to 0.5

7. The process according to any of claims 1 to 6, wherein $M^1$ = Te.

8. The process according to any of claims 1 to 7, wherein at least 50 mol% of the total amount of $M^2$ is Nb.

9. The process according to any of claims 6 to 8, wherein $M^3$ is at least one element from the group consisting of Ni, Co, Pd and Bi.

10. The process according to any of claims 1 to 9, wherein the preparation of the multimetal oxide material M to be washed is effected by a hydrothermal method.

11. A process for the heterogeneously catalyzed gasphase partial oxidation and/or ammoxidation of a saturated and/or unsaturated hydrocarbon, wherein the catalytically active material used is the direct product of a process according to any of claims 1 to 10.

**Revendications**

1. Procédé pour la préparation d'une masse d'oxyde multimétallique M de stoechiométrie générale I

$$Mo_1V_aM^1{}_bM^2{}_cM^3{}_dO_n \qquad (I),$$

où

$M^1$ = au moins un des éléments du groupe comprenant Te et Sb ;
$M^2$ = au moins un des éléments du groupe comprenant Nb, Ti, W, Ta et Ce ;
$M^3$ = au moins un des éléments du groupe comprenant Pb, Ni, Co, Bi, Pd, Ag, Pt, Cu, Au, Ga, Zn, Sn, In, Re, Ir, Sm, Sc, Y, Pr, Nd et Tb ;
a = 0,01 à 1,
b = > 0 à 1,
c = > 0 à 1,
d = > 0 à 0,5 et
n = un nombre qui est déterminé par la valence et la fréquence des éléments différents de l'oxygène dans (I),

dont le diffractogramme de rayons X présente des réflexions de diffraction h, i et k dont les points culminants se situent aux angles de diffraction (2θ) 22,2 ± 0,5° (h), 27,3 ± 0,5° ° (i) et 28,2 ± 0,5° (k), où

- la réflexion de diffraction h dans le diffractogramme de rayons X présente l'intensité la plus forte et une largeur à demi-hauteur d'au plus 0,5°,
- l'intensité $P_i$ de la réflexion de diffraction i et l'intensité $P_k$ de la réflexion de diffraction k répondent à la relation $0,65 \leq R \leq 0,85$ où R est le rapport d'intensités défini par la formule

$$R = P_i / (P_i + P_k)$$

et
- la largeur à demi-hauteur de la réflexion de diffraction i et de la réflexion de diffraction k est à chaque fois ≤ 1°,

mais ne présente pas de réflexion de diffraction présentant une position de point culminant 2θ = 50,0 ± 0,3°, dans lequel on prépare d'abord une telle masse d'oxyde multimétallique M à la condition qu'au cours de la préparation de cette masse d'oxyde multimétallique M, aucune masse d'oxyde multimétallique précurseur de cette masse d'oxyde multimétallique M ne soit lavée avec un liquide du groupe comprenant les membres acides organiques, acides inorganiques, solutions d'acides organiques, solutions d'acides inorganiques et mélanges des membres susmentionnés et qui est **caractérisé en ce que** la masse d'oxyde multimétallique M ainsi préparée est lavée dans un premier temps avec un liquide du groupe comprenant les membres acides organiques, acides inorganiques, solutions d'acides organiques, solutions d'acides inorganiques et mélanges des membres susmentionnés.

2. Procédé selon la revendication 1, **caractérisé en ce que** le liquide avec lequel on lave est une solution aqueuse d'acide nitrique.

3. Procédé selon la revendication 1, **caractérisé en ce que** le diffractogramme de rayons X de la masse d'oxyde multimétallique M à laver contient, outre les réflexions de diffraction h, i et k, encore d'autres réflexions de diffraction, dont les points culminants se trouvent aux angles de diffraction suivants 2θ :

9,0 ± 0,4° (1),
6,7 ± 0,4° (o), et
7,9 ± 0,4° (p).

4. Procédé selon la revendication 3, **caractérisé en ce que** le diffractogramme de rayons X de la masse d'oxyde multimétallique M à laver contient, outre les réflexions de diffraction h, i, k, l, o et p, encore d'autres réflexions de diffraction, dont les points culminants se trouvent aux angles de diffraction suivants 2θ :

45,2 ± 0,4° (q),
29,2 ± 0,4° (m), et
35,4 ± 0,4° (n).

5. Procédé selon la revendication 4, **caractérisé en ce que** le diffractogramme de rayons X de la masse d'oxyde multimétallique M à laver contient les réflexions de diffraction h, i, l, m, n, o, p et q présentant dans la même échelle d'intensité, les intensités suivantes :

h : 100 ;
i : 5 à 95 ;
l : 1 à 30 ;
m : 1 à 40 ;
n : 1 à 40 ;
o : 1 à 30 ;
p : 1 à 30 et
q : 5 à 60.

6. Procédé selon l'une quelconque des revendications 1 à 5, **caractérisé en ce que** les coefficients stoechiométriques a, b, c et d de la masse d'oxyde multimétallique M à laver se trouvent simultanément dans la trame suivante :

EP 1 556 337 B1

a = 0,05 à 6 ;
b = 0,01 à 1 ;
c = 0,01 à 1 ; et
d = 0,00005 à 0,5.

7.  Procédé selon l'une quelconque des revendications 1 à 6, **caractérisé en ce que** $M^1$ = Te.

8.  Procédé selon l'une quelconque des revendications 1 à 7, **caractérisé en ce que** $M^2$ est, à raison d'au moins 50% en mole de sa quantité totale, Nb.

9.  Procédé selon l'une quelconque des revendications 6 à 8, **caractérisé en ce que** $M^3$ est au moins un élément du groupe comprenant Ni, Co, Pd et Bi.

10. Procédé selon l'une quelconque des revendications 1 à 9, **caractérisé en ce que** la préparation de la masse d'oxyde multimétallique M à laver a lieu par voie hydrothermique.

11. Procédé d'oxydation et/ou d'ammoxydation partielle en phase gazeuse catalysée par voie hétérogène d'un hydrocarbure saturé et/ou insaturé, **caractérisé en ce qu'**on utilise comme masse catalytiquement active le produit de procédé direct d'un procédé selon l'une quelconque des revendications 1 à 10.

# FIG.1

# FIG.2

# FIG.3

# FIG.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10248584 A
- DE 10119933 A
- DE 10118814 A
- JP 8057319 A
- DE 19835247 A
- EP 895809 A
- DE 10122027 A
- DE 10051419 A
- DE 10046672 A
- EP 529853 A
- EP 603836 A
- EP 608838 A
- EP 962253 A
- EP 1080784 A
- EP 1090684 A
- EP 1123738 A
- EP 1192987 A
- EP 1192986 A
- EP 1192982 A
- EP 1192983 A
- EP 1192988 A
- DE 10145958 A
- JP 7315842 A
- DE 10029338 A
- JP 2000143244 A
- EP 0204073 W
- DE 2909671 A
- DE 10101695 A
- WO 9822421 A
- DE 10246119 A
- WO 0029106 A
- JP 10036311 A
- EP 1193240 A
- DE 19924532 A
- JP 7053448 A
- DE 2351151 A
- JP 6166668 A
- JP 7232071 A
- JP 6211767 A
- US 4250346 A
- EP 261264 B

**In der Beschreibung aufgeführte Nicht-Patentliteratur**

- *Polyhedron,* 1987, vol. 6 (2), 213-218
- *Anderson Typ bildet Kinetics and Catalysis,* 1999, vol. 40 (3), 401-404